# EUROPÄISCHE PATENTANMELDUNG

(11) **EP 0 864 862 A2**
(43) Veröffentlichungstag der Anmeldung: **16.09.1998**
(21) Anmeldenummer: 98103005.9
(22) Anmeldetag: 20.02.1998
(51) Int. Cl.: G01N 33/00, B01D 5/00

(54) **Kondensatabscheider mit Kondensatabführung**

(30) Priorität: 14.03.1997 DE 19710581
(71) Anmelder: Testo GmbH & Co., 79853 Lenzkirch (DE)
(72) Erfinder: Senn, Jürgen, 79868 Feldberg (DE)
(74) Vertreter: Patentanwälte Westphal, Buchner, Mussgnug Neunert, Göhring

(57) **Zusammenfassung**

Kondensatabscheider, insbesondere für eine transportable (Rauch-)Gasanalyseeinrichtung, mit einem Abscheideraum (26), der im Inneren eines Gehäuses (20) gebildet ist und vom Meßgas durchströmt wird, einem Gaszuführungselement (30), das in den Abscheideraum (26) hineingeführt ist, einem Gasabführungselement (40), das aus dem Abscheideraum (26) herausgeführt ist, einem Kondensatsammelraum (99), der in kommunizierender Verbindung zu dem Abscheideraum (26) vorgesehen ist, wobei der Kondensatsammelraum (99) zumindest teilweise mit flüssigkeitsaufnehmendem Material (100) gefüllt ist, und weiterhin ein Kondensatabführungselement (92) vorgesehen ist, das aus dem Kondensatsammelraum (99) herausgeführt ist.

## Beschreibung

Die Erfindung betrifft einen Kondensatabscheider, insbesondere für eine transportable (Rauch-)Gasanalyseeinrichtung gemäß Oberbegriff des Anspruchs 1.

Zur Anlalyse des Rauchgases von Feuerungsanlagen und des Abgases von beispielsweise Gasmotoren wird Meßgas mittels einer Sonde angesaugt und einem Analysegerät zugeführt. Für die Gasanalyse ist es erforderlich, das Meßgas aufzubereiten, insbesondere müssen Rauch- und Schmutzpartikel sowie Feuchtigkeit aus dem Meßgas entfernt werden.

Es ist deshalb üblich, einen Kondensatabscheider vorzusehen, der die im Meßgas enthaltene Feuchtigkeit praktisch vollständig entfernt. In der Gasanalyseeinrichtung bzw. an den darin enthaltenen Sensoren fällt kein Kondensat mehr an.

Der grundsätzliche Aufbau eines solchen Kondensatabscheiders, wie er insbesondere bei transportablen Gasanalyseeinrichtungen zum Einsatz kommt, ist aus der DE 41 01 194 C1 bekannt. Dieser Kondensatabscheider ist besonders einfach aufgebaut und eignet sich für den mobilen Einsatz.

Bei diesem Kondensatabscheider durchströmt das Meßgas einen Kondensatabscheideraum, der im Inneren eines Gehäuses gebildet und gegenüber der Umgebung abgedichtet ist. Das Gehäuse ist rohrförmig ausgebildet und jeweils endseitig mit Stopfen verschlossen. Die Stopfen nehmen Gasführungselemente in Form von Röhrchen auf, die nach außen hin mit Anschlußnippeln für Schläuche versehen und in den Abscheideraum hinein bzw. aus diesem herausgeführt sind.

Das Meßgas wird zuströmseitig über das Gaszuführungselement in den Abscheideraum hineingeleitet. Dort findet die Abscheidung der im Meßgas enthalteten Feuchtigkeit statt, die beispielsweise durch Mehrfachumlenkung des Meßgasstroms und die damit erzeugten Turbulenzen bewirkt wird. Das auf diese Weise entfeuchtete Meßgas verläßt den Abscheideraum gegenüberliegend durch das Gasabführungselement und wird der Analyseeinrichtung zugeleitet. Das abgeschiedene Kondensat verbleibt im Inneren des Abscheideraums und schlägt sich beispielsweise an der Gehäusewandung nieder. Es ist deshalb erforderlich, das Kondensat in gewissen Abständen von Hand zu entfernen. Hierzu wird einer der beiden Stopfen vom Gehäuse abgezogen, so daß das darin enthaltene Kondensat aus dem Gehäuse ablaufen kann. Je nach anfallender Kondensatmenge kann es erforderlich sein, den Entleervorgang in relativ kurzen Abständen zu wiederholen. Um eine visuelle Kontrolle der Bedienperson zu ermöglichen, ist deshalb das Gehäuse aus einem transparenten Material gefertigt.

Aus der DE 42 41 891 A1, von der die Erfindung ausgeht, ist ein weiterer Kondensatabscheider bekannt, der einen im Inneren eines Gehäuses gebildeten Abscheideraum aufweist. Auch besitzt er ein Gaszuführungselement, das in den Abscheideraum hineingeführt ist, sowie ein Gasabführungselement, das aus dem Ausscheideraum herausgeführt ist. Darüber hinaus besitzt der Kondensatabscheider einen Kondensatsammelraum, der Bestandteil des Abscheideraums ist, der das anfallende Kondensat aufnimmt. Der Kondensatsammelraum ermöglicht eine vereinfachte Bedienung dahingehend, daß jederzeit anhand der im Kondensatsammelraum enthaltenen Kondensatmenge feststellbar ist, ob eine Entleerung notwendig ist. Nach wie vor muß jedoch die Entleerung von Hand vorgenommen werden. Darüber hinaus ist es - ebenso wie bei dem eingangs beschriebenen Kondensatabscheider - unumgänglich, den Meßvorgang zu unterbrechen, um das Kondensat zu entleeren. Dies ist speziell dann problematisch, wenn kontinuierlich über längere Zeiträume hinweg bei gleichzeitig hohem Anfall von Kondensat gemessen werden soll.

Der Erfindung lag daher das Problem zugrunde, einen gattungsgemäßen Kondensatabscheider derart weiterzuentwickeln, daß er die geschilderten Nachteile nicht mehr aufweist. Insbesondere sollte ein Kondensatabscheider geschaffen werden, der eine vollständige Kondensatentleerung ohne Öffnen des Gehäuses zuläßt.

Gelöst wird dieses Problem mit einem Kondensatabscheider, der die Merkmale des Anspruchs 1 aufweist.

Vorteilhafte Ausgestaltungsformen der Erfindung sind Gegenstand der Unteransprüche.

Die Erfindung basiert auf der Idee, flüssigkeitsaufnehmendes Material in den Kondensatsammelraum zu integrieren, und anfallendes Kondensat, das von dem Material aufgenommen ist, durch ein Kondensatabführungselement nach außen abzuleiten. Das flüssigkeitsaufnehmende Material dient hierbei einerseits als Flüssigkeitspuffer, so daß der Entleervorgang mittels einer geeigneten Pumpe nicht kontinuierlich, sondern lediglich in gewissen Abständen erfolgen muß. Andererseits gelingt eine praktisch vollständige Entleerung des im Inneren des Gehäuses befindlichen Kondensats auch dann noch, wenn das Aufnahmevermögen des im Kondensatsammelraum angeordneten Materials erschöpft sein sollte und deshalb weiteres Kondensat im Abscheideraum ungebunden niedergeschlagen ist. Soweit bei einem derartigen Betriebszustand durch Initiieren des Absaugevorgangs Kondensat aus dem flüssigkeitsaufnehmenden Material durch das Kondensatabführungselement abgezogen wird, werden die zunächst noch frei im Abscheideraum befindlichen Kondensatbestandteile nach und nach vom flüssigkeitsaufnehmenden Material erfaßt und anschließend ebenfalls abgeführt.

Die Wirkungsweise des erfindungsgemäßen Kondensatabscheiders beruht demnach auf dem Kapillareffekt, der für das flüssigkeitsaufnehmende Material typisch ist.

Bevorzugt sind der Kondensatsammelraum und das Kondensatabführungselement Bestandteile eines oder auch von zwei Stopfen, mit denen ein rohrförmiges Gehäuse jeweils endseitig verschlossen ist. Auf diese Weise ist es möglich, den eingangs beschriebenen und bestens bewährten Kondensatabscheider einschließlich vergleichbar aufgebauter Weiterentwicklungen mit dem erfindungsgemäßen Konzept der Kondensatbindung und -abführung zu versehen, ohne den Aufbau im übrigen zu verändern. Damit eröffnet sich auch die Möglichkeit, vorhandene Kondensatabscheider herkömmlicher Bauart durch Austausch der Stopfen umzurüsten.

Bevorzugt sind zwei einander gegenüberliegende Kondensatsammelräume vorgesehen, die jeweils flüssigkeitsaufnehmendes Material enthalten. Damit ist es möglich, einen lageunabhängigen Betrieb des Kondensatabscheiders zu realisieren. Auch Lageänderungen während des Betriebs beeinträchtigen die Funktionssicherheit nicht, da das Kondensat je nach aktueller Winkellage bei beliebigen Abweichungen aus der Horizontalen zwangsweise dem einen oder dem anderen Kondensatsammelraum zugeführt wird.

Diese Variante läßt sich äußerst kostengünstig durch zwei übereinstimmend ausgebildete Stopfen realisieren, die jeweils einen Kondensatsammelraum und ein Kondensatabführungselement enthalten. Es versteht sich von selbst, daß in diesem Falle an beiden Kondensatabführungselementen Kondensat abgepumpt werden muß. Dies kann beispielsweise bei Verwendung einer einfachen Schlauchpumpe durch ein Umschaltventil erfolgen, so daß hintereinander bzw. im Wechsel beide Kondensatsammelräume abgepumpt werden. Alternativ ist es auch möglich, eine Schlauchpumpe mit doppeltem Anschluß zu verwenden.

Besonders bevorzugt ist jedoch die Variante, bei der eine flüssigkeitstransportierende Verbindung zwischen den beiden Kondensatsammelräumen hergestellt ist. Weiteres flüssigkeitsaufnehmendes Material ist vorzugsweise innerhalb eines zwischen den beiden Kondensatsammelräumen verlaufenden Verbindungsrohres angeordnet. Hier genügt das Abziehen von Kondensat durch ein einziges Kondensatabführungselement, da infolge der Kapillarwirkung des Materials auch Kondensat aus dem gegenüberliegenden Kondensatsammelraum abgezogen wird.

Weitere bevorzugte Varianten sind auf die konkrete Ausgestaltung des Kondensatsammelraums in Verbindung mit den die Gasführungselemente enthaltenden Stopfen gerichtet.

Bevorzugt ist der Kondensatsammelraum als Ringraum zwischen der ringförmigen Außenwandung des Stopfens und dem Gasführungselement, das den Stopfen axial durchsetzt, gebildet. Hierdurch wird es möglich, den Kondensatsammelraum ohne Vergrößerung des Platzbedarfs gegenüber den Stopfen aus dem Stand der Technik zu integrieren.

Vorteilhafterweise ist der Kondensatsammelraum axial nach außen hin durch eine scheibenförmige Abdeckung verschlossen, an der das Kondensatabführungselement integral angeformt ist. Damit wird ohne großen Bauaufwand die Anschlußmöglichkeit für den zur Pumpe führenden Schlauch geschaffen. Durch die stirnseitige Anordnung an dem Kondensatabscheider ist der Anschluß einerseits gut zugänglich, andererseits vergrößert sich das Bauvolumen des Kondensatabscheiders insgesamt kaum.

Fertigungstechnisch ist es von Vorteil, die Abdeckung in eine korrespondierende Absatzbohrung des Stopfens dicht einzupressen. Diese Art der Verbindungstechnik ermöglicht die kostengünstige Herstellung eines Stopfens, der nach dem Einlegen des flüssigkeitsaufnehmenden Materials und dem anschließenden Einpressen der Abdeckung als vorgefertigte Baugruppe kostengünstig zu montieren ist.

Die kommunizierende Verbindung zwischen dem Abscheideraum und dem Kondensatsammelraum wird auf einfache Weise dadurch realisiert, daß zumindest eine, vorzugsweise mehrere, gleichmäßig über dem Umfang verteilte Bohrungen vorgesehen sind. Die Bohrungen schwächen einerseits die Struktur des Stopfens nicht nennenswert, andererseits sind sie ausreichend, um das während des Betriebs anfallende Kondensat zum Kondensatsammelraum hindurchzuleiten.

Bevorzugt wird als flüssigkeitsaufnehmendes Material ein saugfähiges Vliesmaterial verwendet, vorzugsweise solches auf der Basis von Viskose. Dieses Material ist kostengünstig als Massenware erhältlich und läßt sich besonders gut verarbeiten. Darüber hinaus besitzt es ein ausgezeichnetes Aufnahmevermögen für Flüssigkeiten bei gleichzeitig hoher Kapillarwirkung. Besonders günstig ist die Eigenschaft, daß sich das Material bereits bei vergleichsweise geringer Feuchtigkeitsaufnahme ausdehnt und somit eine gewisse Dichtungswirkung insbesondere im Bereich des Kondensatabführungselements entfaltet. Eine solche Dichtungswirkung ist insbesondere auch deshalb erwünscht, um Leckgasströme weitgehend zu vermeiden.

Besondere Vorteile ergeben sich dann, wenn der Kondensatabscheider mit einer Schlauchpumpe entleert wird. Diese hat die vorteilhafte Eigenschaft, daß sie stets eine dichte Funktion auf den stromaufwärts gerichteten Schlauchabschnitt ausübt, somit eine gegenüber der Umgebung abgedichtete Ausführung des Abscheideraums einschließlich der Kondensatabführungsbaugruppe zuläßt.

Die Erfindung wird nachstehend näher anhand der in den Figuren dargestellten Ausführungsbeispiele erläutert. Es zeigen:
- Fig. 1: Axialschnitt durch einen Kondensatabscheider,
- Fig. 2: Einzeldarstellung eines Stopfens,
- Fig. 3: Kondensatabscheider, einseitige Kondensatabführung mit einfach wirkender Schlauchpumpe,
- Fig. 4: Kondensatabscheider, zweiseitige Kondensatabführung mit doppelt wirkender Schlauchpumpe,
- Fig. 5: Kondensatabscheider mit Verbindungsrohr, einseitige Kondensatabführung mit einfach wirkender Schlauchpumpe,
- Fig. 6: Kondensatabscheider, zweiseitige Kondensatabführung mit einfach wirkender Schlauchpumpe und Umschaltventil.

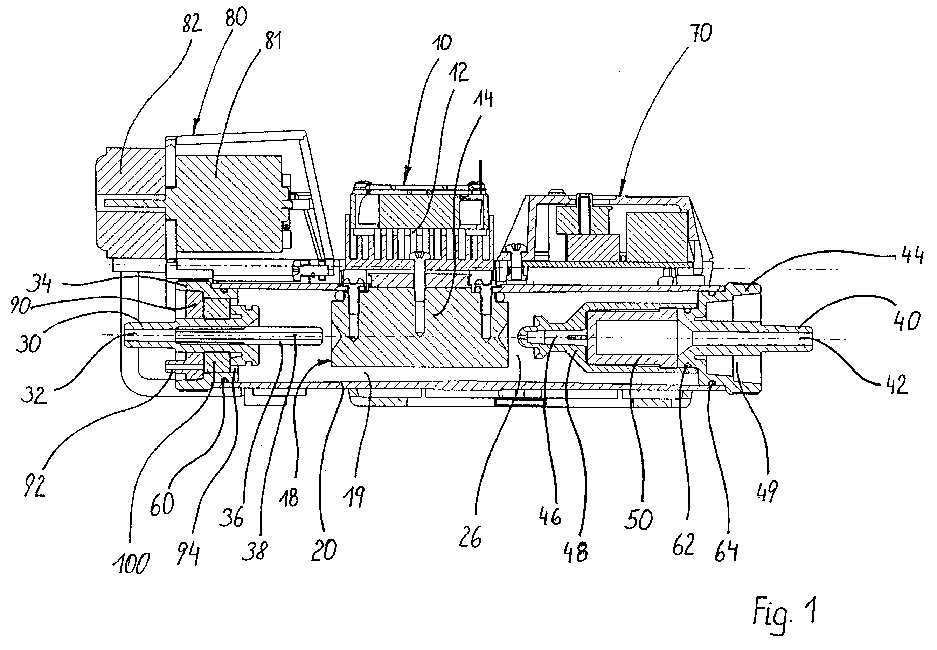

Der in Fig. 1 dargestellte Kondensatabscheider wird lediglich hinsichtlich der erfindungsrelevanten Merkmale detailliert erläutert.

Der Kondensatabscheider besitzt ein rohrförmiges Gehäuse 20, welches zur Bildung eines Abscheideraums 26 jeweils endseitig gegenüberliegend mit Stopfen 34, 44 verschlossen ist. Zwischen den Stopfen 34, 44 sind Dichtungsringe 60, 64 vorgesehen, die einen dichten Abschluß zwischen den genannten Bauteilen sicherstellen.

Am Stopfen 34 ist ein rohrförmiges Gaszuführungselement 30 integral angeformt, welches in axialer Richtung vollständig von einer Bohrung 32 durchsetzt ist. Am Gaszuführungselement 30 ist ein nicht dargestellter Meßgasschlauch mit endseitig angebrachter Entnahmesonde anschließbar.

Gegenüberliegend ist am Stopfen 44 ein rohrförmiges Gasabführungselement 40 angeformt, das in axialer Richtung vollständig von einr Bohrung 42 durchsetzt ist. Am Gasabführungselement 40 ist eine hier nicht näher dargestellte Leitung zur Gasanalyseeinrichtung anschließbar.

Durch das Rohr 20 ist ein Peltier-Element 10 hindurchgeführt, das in an sich bekannter Weise eine Warmseite 12 besitzt, die außerhalb des Rohres 20 angeordnet ist und der Wärmeabführung dient.

Innerhalb des Rohres 20 ist eine Kaltseite 14 angebracht, die praktisch vollständig in dem Abscheideraums 26 hineinragt und unmittelbar als Wärmetauscherfläche für das hindurchströmende Meßgas konzipiert ist. Zwischen der Kaltseite 14 und dem Gehäuse 20 verbleibt ein Radialspalt 19.

Zur weiteren Verbesserung der Kondensatabscheidung werden zusätzlich Strömungsumlenkungen erzwungen. Hierzu trägt der Stopfen 34 ein mit einer axialen Durchgangsbohrung 38 versehenes Zapfenelement 36, das konzentrisch zum Rohr 20 verläuft und axial dicht an die Kaltseite 14 herangeführt ist. Das stirnseitig aus dem Zapfenelement 36 aus der Bohrung 38 austretende Meßgas prallt somit auf die Stirnseite 18 der Kaltseite 14 auf. Die Stirnseite 18 hat damit die Funktion einer Prallfläche. Infolge des Aufpralls wird das Meßgas zunächst entgegen der Auftreffrichtung umgelenkt, bevor es nach einer erneuten Umlenkung durch den Axialspalt 19 durchgeleitet wird und an der Kaltseite 14 entlang geführt wird.

Hinter der Kaltseite 14 erfolgt erneut eine zweifache Umlenkung, die durch ein weiteres Zapfenelement 46 bewirkt wird, das vom Stopfen 44 getragen ist. Es weist wiederum eine Bohrung 48 auf, die koaxial zum Rohr 20 verläuft. Das Zapfenelement 46 ist ebenfalls bis dicht an die Kaltseite 14 herangeführt, so daß der Meßgasstrom zur Richtungsänderung gezwungen ist, um in die Bohrung 48 eintreten zu können. Die Bohrung 48 mündet im Inneren des Zapfenelements 46, das gegenüber dem Stopfen 44 mittels eines Dichtungsrings 62 abgedichtet ist und einen Filter 50 zur Abscheidung von Partikeln aufnimmt.

Als Abscheideraum 26 steht demnach der nicht verbaute Innenraumanteil des Rohres 20 zur Verfügung.

Außen am Gehäuse 20 ist eine Elektronik-Baugruppe 70 angebracht, die in hier nicht näher interessierender Art und Weise Betriebszustände anzeigt und/oder steuert.

Das im Abscheideraum 26 anfallende Kondensat wird über ein Kondensatabführungselement 92 aus dem Inneren des Gehäuses 20 heraus abtransportiert. Das Kondensatabführungselement 92 ist als Schlauchnippel ausgebildet, an den ein Schlauch 84 aufsteckbar und an eine Schlauchpumpe 80, bestehend aus einem Antriebsmotor 81 und Schlauchkassette 82, anschließbar ist. Die Schlauchpumpe 80 ist zur Realisierung einer besonders kompakten Bauweise außen am Gehäuse 20 befestigt.

Im Bereich des Stopfens 34 ist ein Kondensatsammelraum 99 als Ringraum zwischen der ringförmigen Außenwandung des Stopfens 34 und dem den Stopfen 34 axial durchsetzenden Gasführungselement 30 gebildet. Er ist damit Bestandteil einer ringförmigen Absatzausnehmung 39, die von außen am Stopfen 34 eingeformt ist.

Der Kondensatsammelraum 99 ist praktisch vollständig mit einem Viskosevlies gefüllt, das eine hohe Kapillarwirkung und ein hohes Aufnahmevermögen für Flüssigkeiten besitzt.

Nach außen hin ist der Kondensatsammelraum 99 durch eine scheibenförmige Abdeckung 90 verschlossen, die in den Stopfen 34, und zwar in die Absatzausnehmung 39 dicht eingepreßt ist. Der Schlauchnippel 92 ist integral an der Abdeckung 90 angeformt.

Zur Bildung einer kommunizierenden Verbindung zwischen dem Abscheideraum 26 und dem Kondensatsammelraum 99 sind stirnseitig am Stopfen 34 acht gleichmäßig über dem Umfang verteilte Bohrungen 94 angebracht. Durch die Bohrungen 94 wird anfallendes Kondensat infolge der Kapillarwirkung des Vlieses 100 in den Kondensatsammelraum 99 hineingezogen und vom Vlies 100 aufgenommen. Der Abtransport des Kondensats erfolgt in gewissen Abständen, die sich im wesentlichen nach der anfallenden Kondensatmenge richten. Hierzu wird die Schlauchpumpe 80, beispielsweise automatisch gesteuert, in Betrieb gesetzt.

Bei diesem Kondensatabscheider erfolgt die Abführung des Kondensats einseitig, d.h. im Bereich der Zuführung des Meßgases. Aufgrund der Anordnung ist eine Kondensatabführung dann möglich, wenn der Kondensatabscheider zumindest geringfügig abweichend von der Horizontalen zu derjenigen Seite geneigt gehalten ist, an der sich der Kondensatsammelraum 99 befindet. Dies erleichtert das Abfließen von Kondensat in Richtung auf den Kondensatsammelraum 99. Die Winkellage in Bezug auf die Längsachse (Rotationsachse) ist unbeachtlich, da durch die Vielzahl der auf dem Umfang angeordneten Bohrungen 94 in jedem Fall ein unmittelbarer Zufluß zum Kondensatsammelraum 99 möglich ist.

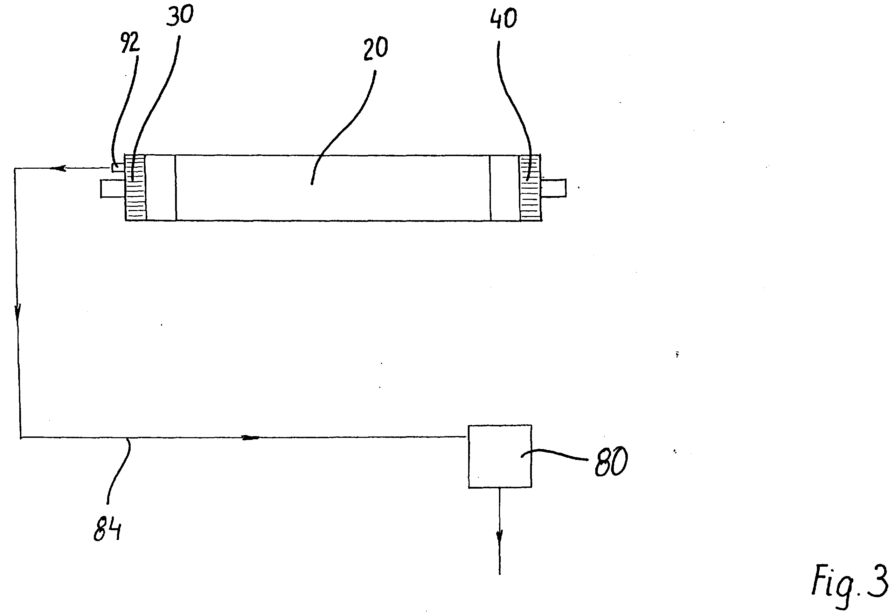

Die grundsätzliche Konzeption einer einseitigen Kondensatabführung mit einer einfach wirkenden Schlauchpumpe 80 ist nochmals in Figur 3 verdeutlicht. Sie zeigt das Gehäuse 20, welches jeweils endseitig mit den Stopfen 30, 40 verschlossen ist. Lediglich einer der beiden Stopfen, nämlich Stopfen 30 weist einen Schlauchnippel 92 auf, an dem der Schlauch 84 angeschlossen ist, welcher die Verbindung zur Schlauchpumpe 80 herstellt.

Sofern die Realisierung einer vollkommen lageunabhängigen Kondensatabführung gewünscht wird, ist es erforderlich, einen weiteren Kondensatsammelraum 99 im Bereich des Stopfens 44 vorzusehen. Der Stopfen 44 kann grundsätzlich identisch zum Stopfen 30 ausgeführt sein, sofern auf den Filter 50 verzichtet werden kann. Auch ist es ohne weiteres möglich, den Stopfen 44 so zu modifizieren, daß er ebenfalls zur Kondensataufnahme verwendet werden kann. Er besitzt bereits eine ringförmige Absatzausnehmung 49, die in Übereinstimmung mit der Absatzausnehmung 39 des Stopfens 34 gestaltet ist. In die Absatzausnehmung 49 läßt sich ebenso ein weiteres Vlies 100 einsetzen und eine Abdeckung 90 dicht einPressen. Als zusätzliche Maßnahme müssen lediglich in geeigneter Weise Durchgangsbohrungen angebracht werden, um die Verbindung zum Abscheideraum 26 herzustellen.

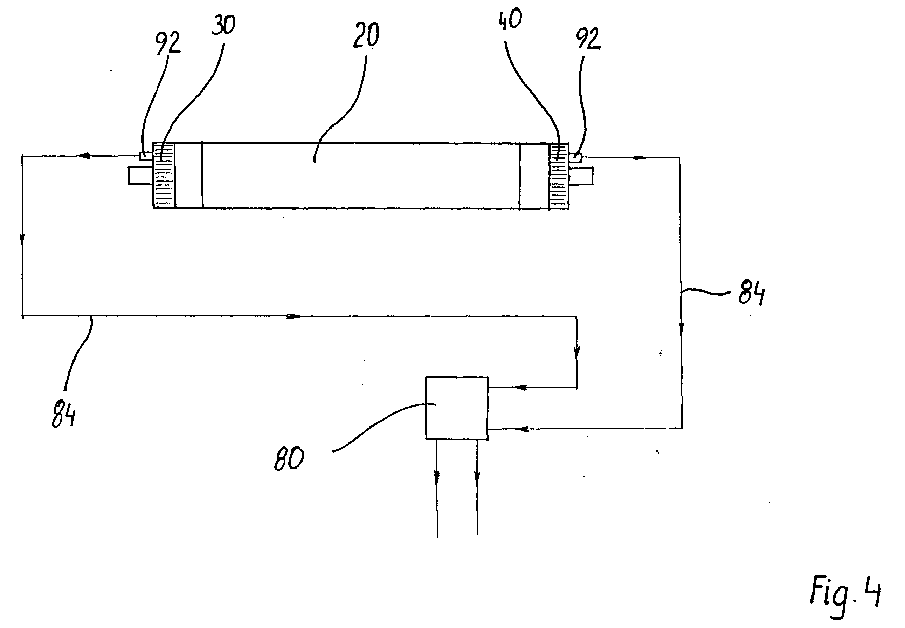

Das Abführen von Kondensat kann nun auf zwei unterschiedliche Arten erfolgen. Gemäß der in Figur 4 dargestellten Variante ist auch im Bereich des Gasabführungselements 40 ein Schlauchnippel 92 vorgesehen, an den ebenfalls ein Schlauch 84 angeschlossen ist. Weiterhin ist die Schlauchpumpe 80 doppelwirkend ausgeführt, d.h. beide Schläuche 84 werden im Parallelbetrieb bedient, so daß eine gleichzeitige Entleerung der beiden Kondensatsammelräume 99 erfolgt.

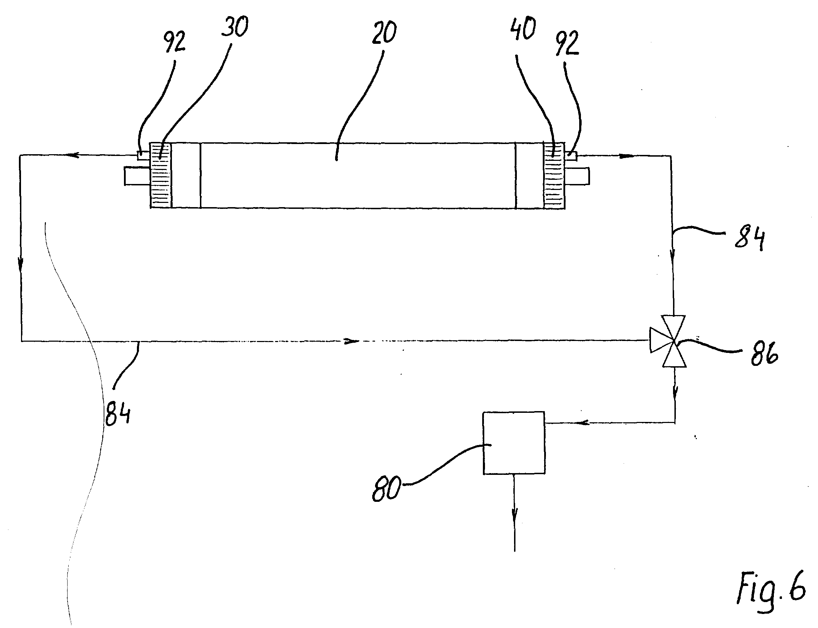

Alternativ hierzu kann weiterhin eine einfachwirkende Schlauchpumpe 80 verwendet werden, sofern die beiden Schläuche 84 an einem Umschaltventil 86 zusammengeführt sind. Diese Variante ist in Figur 6 dargestellt.

Diese Variante erlaubt weiterhin die Verwendung einer einfachwirkenden Schlauchpumpe, wobei die Entleerung der beiden Kondensatsammelräume 99 durch Umschalten des Ventils 86 zeitlich nacheinander bzw. im Wechsel erfolgt.

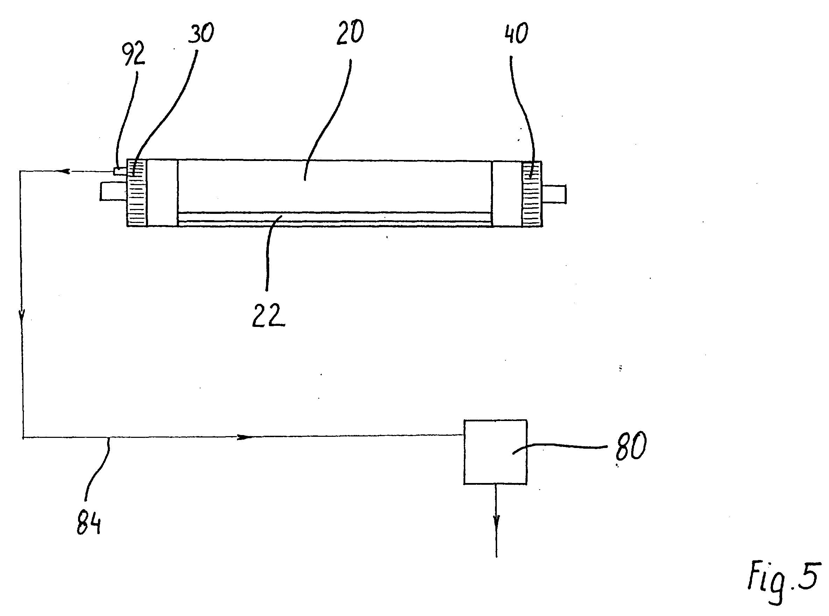

Besonders vorteilhaft ist deshalb die in Figur 5 dargestellte Variante, bei der weiterhin eine einfachwirkende Schlauchpumpe 80 vorgesehen ist, die Kondensat lediglich einseitig, beispielsweise im Bereich des Gaszuführungselements 30 absaugt. Der lageunabhängige Betrieb wird in diesem Fall dadurch erreicht, daß ein Verbindungsrohr 22 innerhalb des Gehäuses 20 vorgesehen ist, welches die beiden Kondensatsammelräume 99 miteinander verbindet. Das Verbindungsrohr 22 ist ebenfalls mit Vliesmaterial gefüllt, so daß eine durchgehende flüssigkeitstransportierende Verbindung von dem dem Gasabführungselement 40 zugeordneten Kondensatsammelraum 99 zu dem gegenüberliegenden Kondensatsammelraum 99 hergestellt ist, über den die Kondensatabführung erfolgt. Es versteht sich von selbst, daß in diesem Fall eine Abdeckung 90 verwendet werden muß, die keinen Anschlußnippel 92 aufweist. Alternativ ist es auch möglich, einen ggfs. vorhandenen Anschlußnippel 92 dicht zu verschließen.

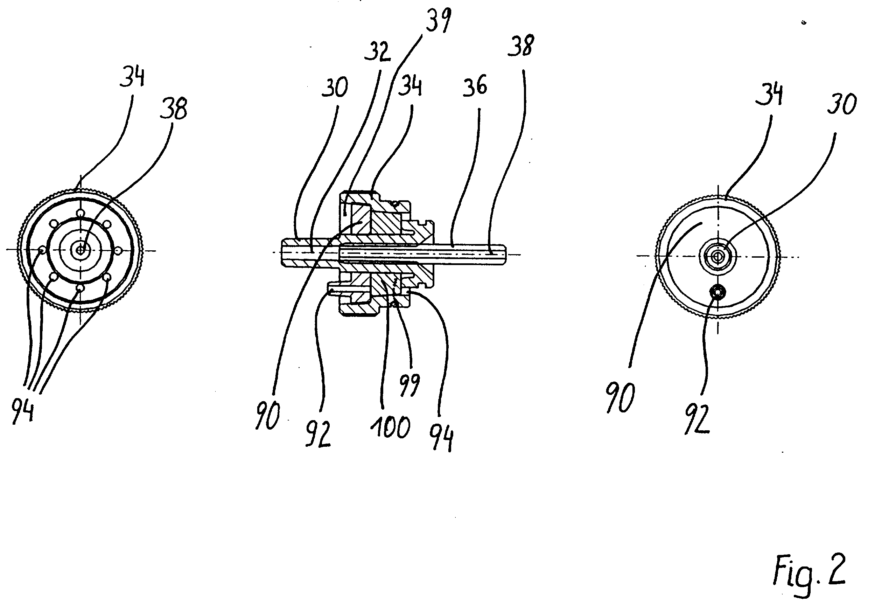

Aus dem vorstehenden ergibt sich, daß durch vergleichsweise einfache Modifikationen im Bereich der Stopfen 34 und/oder 44 eine komfortable Kondensatabführung aus dem Kondensatabscheider realisiert werden kann. Durch simplen Austausch eines Stopfens, wie er beispielsweise in Figur 2 dargestellt ist, kann ein herkömmlicher Kondensatabscheider umgerüstet werden.

### Bezugszeichenliste

- 10: Peltier-Element
- 12: Warmseite
- 14: Kaltseite
- 18: Prallfläche
- 19: Radialspalt
- 20: Rohr
- 22: Verbindungsrohr
- 26: Abscheideraum
- 30: Gaszuführungselement
- 32: Bohrung
- 34: Stopfen
- 36: Zapfenelement
- 38: Bohrung
- 39: Absatzausnehmung
- 40: Gasabführungselement
- 42: Bohrung
- 44: Stopfen
- 46: Zapfenelement
- 48: Bohrung
- 49: Absatzausnehmung
- 50: Filter
- 60: Dichtungsring
- 62: Dichtungsring
- 64: Dichtungsring
- 70: Elektronik-Baugruppe
- 80: Schlauchpumpe
- 81: Antriebsmotor
- 82: Schlauchkassette
- 84: Schlauch
- 86: Umschaltventil
- 90: Abdeckung
- 92: Schlauchnippel
- 94: Bohrung
- 99: Kondensatsammelraum
- 100: Vlies

## Patentansprüche

1. Kondensatabscheider, insbesondere für eine transportable (Rauch-)Gasanalyseeinrichtung, mit
- einem Abscheideraum (26), der im Inneren eines Gehäuses (20) gebildet ist und vom Meßgas durchströmt wird,
- einem Gaszuführungselement (30), das in den Abscheideraum (26) hineingeführt ist,
- einem Gasabführungselement (40), das aus dem Abscheideraum (26) herausgeführt ist,
- einem Kondensatsammelraum (99), der in kommunizierender Verbindung zu dem Abscheideraum (26) vorgesehen ist,
dadurch gekennzeichnet,
- daß der Kondensatsammelraum (99) zumindest teilweise mit flüssigkeitsaufnehmendem Material (100) gefüllt ist, und
- daß ein Kondensatabführungselement (92) vorgesehen ist, das aus dem Kondensatsammelraum (99) herausgeführt ist.

2. Kondensatabscheider nach Anspruch 1, dadurch gekennzeichnet,
- daß das Gehäuse (20) rohrförmig ausgebildet und jeweils endseitig mit Stopfen (34, 44) verschlossen ist, die jeweils das Gaszuführungselement (30) und das Gasabführungselement (40) aufnehmen, und
- daß der Kondensatsammelraum (99) und das Kondensatabführungselement (92) Bestandteile zumindest eines der beiden Stopfen (34,44) sind.

3. Kondensatabscheider nach Anspruch 1 oder 2, dadurch gekennzeichnet, daß zwei einander gegenüberliegende Kondensatsammelräume (99) vorgesehen sind, die jeweils flüssigkeitsaufnehmendes Material (100) enthalten.

4. Kondensatabscheider nach Anspruch 3, dadurch gekennzeichnet, daß zur Bildung einer flüssigkeitstransportierenden Verbindung zwischen den beiden Kondensatsammelräumen (99) weiteres flüssigkeitsaufnehmendes Material (100) durchgehend, vorzugsweise innerhalb eines Verbindungsrohres (22), angeordnet ist.

5. Kondensatabscheider nach einem der Ansprüche 2 bis 4, dadurch gekennzeichnet, daß der Kondensatabscheideraum (99) als Ringraum zwischen der ringförmigen Außenwandung des Stopfens (34, 44) und dem den Stopfen (34, 44) axial durchsetzenden Gaszuführungselement (30) bzw. Gasabführungselement (40) gebildet ist.

6. Kondensatabscheider nach Anspruch 5, dadurch gekennzeichnet, daß der Kondensatsammelraum (99) axial nach außen hin durch eine scheibenförmige Abdeckung (90) verschlossen ist, an der das Kondensatabführungselement (92) integral angeformt ist.

7. Kondensatabscheideraum nach Anspruch 6, dadurch gekennzeichnet, daß die Abdeckung (90) in eine korrespondierende Absatzbohrung (39, 49) des Stopfens (30, 40) dicht eingepreßt ist.

8. Kondensatabscheider nach einem der Ansprüche 5 bis 7, dadurch gekennzeichnet, daß zur Bildung der kommunizierenden Verbindung zwischen dem Abscheideraum (26) und dem Kondensatsammelraum (99) der Stopfen (34) stirnseitig zumindest eine, vorzugsweise mehrere, gleichmäßige über dem Umfang verteilte Bohrungen (94) aufweist.

9. Kondensatabscheider nach einem der vorstehenden Ansprüche, dadurch gekennzeichnet, daß das flüssigkeitsaufnehmende Material (100) ein saugfähiges Vliesmaterial, vorzugsweise auf der Basis von Viskose, ist.

10. Kondensatabscheider nach einem der vorstehenden Ansprüche, gekennzeichnet durch eine mit dem wenigstens einen Kondensatabführungselement (92) verbundene Schlauchpumpe (80).
